# FASCICULE DE BREVET EUROPEEN

(11) **EP 0 737 199 B1**
(45) Date de publication et mention de la délivrance du brevet: **13.02.2002**
(21) Numéro de dépôt: 95933458.2
(22) Date de dépôt: 29.09.1995
(51) Int. Cl.: C07F 7/08, C07F 7/18, A61K 7/06, A61K 7/09, A61K 31/695

(54) **PROCEDE DE PREPARATION DE COMPOSES DE SILICIUM BIOLOGIQUEMENT ACTIFS SOUS FORME CONCENTREE**
VERFAHREN ZUR HERSTELLUNG VON BIOLOGISCH AKTIVEN SILIZIUMVERBINDUNGEN IN KONZENTRIERTER FORM
METHOD FOR PREPARING CONCENTRATED BIOLOGICALLY ACTIVE SILICON COMPOUNDS

(30) Priorité: 30.09.1994 FR 9412089
(43) Date de publication de la demande: 16.10.1996
(73) Titulaire: EXSYMOL, 98000 Monaco (MC)
(72) Inventeur: SEGUIN, Marie-Christine, MC-98000 Monaco (MC); GUEYNE, Jean, MC-98000 Monaco (MC); NICOLAY, Jean-François, F-06230 Villefranche-sur-Mer (FR); FRANCO, André, F-06500 Menton (FR)
(74) Mandataire: Bonneau, Gérard
(86) Numéro de dépôt international: FR9501267
(87) Numéro de publication internationale: WO9610574

(56) Documents cités:
- EP-A- 0 281 435
- EP-A- 0 295 983
- FR-A- 2 645 863
- GB-A- 827 419
- CHEMICAL ABSTRACTS, vol. 88, no. 5, 30 Janvier 1978 Columbus, Ohio, US; abstract no. 37959, GUEYNE, C.H.J. ET AL. 'BIOLOGICALL ACTIVE, WATER-SOLUBLE SILANES' & BR,A,7 604 340 (GUEYNE, C.H.J. ET AL.)

## Description

### Domaine technique

La présente invention concerne les composés à base de silicium biologiquement actifs et en particulier un procédé de préparation de composés de silicium biologiquement actifs sous forme concentrée à partir de précurseurs hydrolysables.

### Etat de la technique

Le silicium, élément très répandu dans la nature est généralement connu sous ses formes inorganiques naturelles telles que la silice et les silicates, ou encore sous la forme de polymères synthétiques, les silicones. Ces composés siliciés sont très peu ou pas solubles en milieu aqueux ce qui explique leur faible incidence au niveau des organismes vivants. Les silicones, en particulier, se caractérisent par une grande inertie vis à vis des milieux biologiques et par conséquent présentent une biocompatibilité élevée.

Pourtant, le silicium, même à l'état de traces, joue un rôle biologique important et doit être considéré comme un élément essentiel de la vie. Il est notamment nécessaire à une croissance normale pour de nombreuses espèces. Il a aussi été démontré que le silicium intervenait dans la structuration des tissus conjonctifs en interagissant avec les glycosaminoglycanes et les protéines. C'est un des éléments constitutifs des complexes protéines-glycosaminoglycanes rencontrés dans la substance fondamentale de ces tissus. Le silicium interagit également avec les glycosaminoglycanes dans le développement des tissus cartilagineux. On sait aussi que le silicium joue un rôle important dans l'ossification où il favorise le processus de minéralisation.

En outre, le silicium peut être considéré comme un constituant du collagène et on pense qu'il joue un rôle fondamental dans le processus de réticulation des fibres de collagène. Le silicium intervient également au niveau de la structure du cheveu, où il contribuerait notamment à augmenter la résistance de la tige pilaire. Le silicium est également impliqué dans le métabolisme cellulaire et il serait notamment propice à l'activité métabolique des ostéoblastes.

Au delà du pouvoir réticulant du silicium et de son implication dans l'activité métabolique de certaines cellules, il apparaît qu'une teneur élevée en silicium dans les tissus, parallèlement avec la teneur en glycosaminoglycanes, est caractéristique des tissus sains et métaboliquement actifs. De même, de nombreux travaux ont montré l'importance du silicium dans la régulation du cycle physiologique du cheveu.

Les recherches actuelles tendent à renforcer l'idée que le silicium intervient dans de nombreux mécanismes biologiques. Des travaux récents ont même montré que le silicium joue un rôle majeur dans l'élimination de l'aluminium par les systèmes biologiques.

Les travaux de la demanderesse ont montré que les composés siliciés pouvaient constituer une forme de silicium assimilable par l'organisme (par opposition au silicium minéral ou aux silicones) à condition de posséder la propriété d'exister en solution aqueuse sous forme d'oligomères solubles de faible poids moléculaire. En outre, une autre caractéristique nécessaire à l'activité de ces oligomères en solution aqueuse est de présenter de nombreuses fonctions Si-OH. Il ressort ainsi que les propriétés biologiques de ces composés siliciés assimilables par l'organisme, ne sont observées que s'ils forment en solution des oligomères solubles, résultant d'un enchaînement de liaisons siloxanes Si-O-Si, riches en fonctions Si-OH.

En dehors du fait que la présence des fonctions Si-OH, très polaires, confèrent aux oligomères leur solubilité dans l'eau, on pense à l'heure actuelle qu'une partie des propriétés observées s'expliquent par le fait que l'espèce chimique impliquée dans la plupart des mécanismes biologiques mentionnés ci-dessus serait une forme soluble du silicium, l'acide silicique, de formule Si(OH)₄. Mais, étant donnée sa très forte propension à se polycondenser pour former de la silice, ce composé n'existe qu'à de très faibles concentrations dans l'eau.

On a donc recherché des produits analogues à l'acide silicique, plus stables, en modifiant chimiquement les fonctions Si-OH. Mais il est vite apparu que ces fonctions étaient essentielles à l'activité biologique. Par ailleurs, on savait qu'une série de composés naturels, les tanins et les catécholamines entre autres, étaient capables de complexer l'acide silicique et ainsi d'augmenter sa stabilité en solution. Ces complexes constitueraient la forme de transport de l'acide silicique dans l'organisme et c'est sous cette forme que la cellule importerait le silicium. Toutefois, leur stabilité est encore trop faible pour la réalisation d'un produit pharmacologiquement actif.

La demanderesse a mis au point des analogues actifs de ces complexes. Il s'agit des produits résultant de la complexation entre une molécule complexante et un composé organo-silicié actif. La caractéristique de ces composés est de posséder plusieurs fonctions Si-OH comme l'acide silicique, mais également une ou deux liaisons silicium-carbone. Toutefois, ces analogues possédant les propriétés biologiques recherchées, s'ils sont beaucoup plus stables que les complexes d'acide silicique, doivent être préparés sous la forme de solutions aqueuses, diluées, dont la teneur en silicium ne peut pas excéder 2g/litre du fait qu'avec une concentration plus élevée, on favoriserait la polycondensation.

### Exposé de l'invention

C'est pourquoi le but de l'invention est d'obtenir des composés à base de silicium biologiquement actifs n'évoluant pas vers la formation de formes polycondensées inactives telles que des polysiloxanes.

Un autre but de l'invention est de réaliser un procédé de préparation de composés de silicium biologiquement actifs sous forme concentrée par hydrolyse de précurseurs à base de silicium avec libération de produits stabilisants empêchant la polycondensation des composés de silicium.

L'objet de l'invention est donc un procédé de préparation d'un composé de silicium biologiquement actif dans lequel on hydrolyse un précurseur de formule générale: 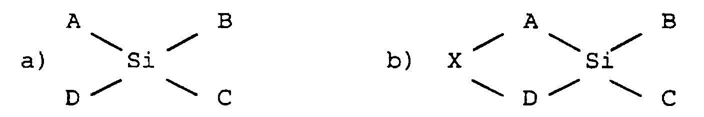 dans laquelle A, B, C, D peuvent être de l'hydrogène, et X dans laquelle A, B, C, D et désignent des radicaux différents de OH et 2 ou 3 de ces radicaux sont hydrolysables ; les radicaux qui sont hydrolysables étant des radicaux liés à Si par un atome d'oxygène ou par un atome d'azote ou de soufre, notamment des esters ou des radicaux alkoxy, aryloxy tels que des radicaux phénoxy ou des radicaux allyloxy ou vinyloxy lorsque l'atome de silicium est lié directement à un atome d'oxygène; soit des thioesters ou des radicaux aryl ou alkylthioéthers lorsque le silicium est lié par un atome de soufre; soit des amines mono ou disubstituées par des chaînes hydrocarbonées substituées ou non par un ou plusieurs groupements fonctionnels, des aryl ou alkylamides lorsque le silicium est lié par un atome d'azote ; les radicaux qui ne sont pas hydrolysables étant des radicaux hydrocarbonés, substitués ou non par un ou plusieurs groupements fonctionnels, certains radicaux alkoxy stériquement encombrés, les radicaux fluorocarbonés, ou même un atome de fluor ; et X étant un radical hydrocarboné, substitué par un ou plusieurs groupements fonctionnels, relié au silicium (liaison A ou D) par au plus une liaison non hydrolysable ;
l'hydrolyse étant effectuée dans un solvant contenant une faible quantité d'eau dans une proportion par rapport au solvant comprise de préférence entre 0,1% et 5% ; et
au moins un des composés provenant des liaisons hydrolysées du silicium étant un composé dit stabilisant empêchant la formation de polymères à partir des liaisons hydrolysées du silicium.

### Description de l'invention

Dans toute la description, on appellera "silyl" le composé biologiquement actif obtenu par le procédé de l'invention, en gardant le mot "précurseur" pour désigner le composé à base de silicium, biologiquement inactif, et hydrolysable selon le procédé de l'invention.

Les radicaux qui sont hydrolysables sont de préférence des radicaux liés à Si par un atome d'oxygène ou par un atome d'azote ou de soufre. Ils peuvent donc être des esters ou des radicaux alkoxy, aryloxy tels que des radicaux phénoxy, ou encore des radicaux allyloxy ou vinyloxy, lorsque l'atome de silicium est lié directement à un atome d'oxygène. Ces radicaux peuvent être également des thioesters ou des radicaux aryl ou alkylthioéthers, lorsque le silicium est lié par un atome de soufre. Les radicaux peuvent encore être des amines mono ou disubstituées par des chaînes hydrocarbonées substituées ou non par un ou plusieurs groupements fonctionnels, des aryl ou alkylamides, lorsque le silicium est lié par un atome d'azote.

Les radicaux liés à Si et qui ne sont pas hydrolysables peuvent être des radicaux hydrocarbonés, substitués ou non par un ou plusieurs groupements fonctionnels, certains radicaux alkoxy stériquement encombrés,-les radicaux fluorocarbonés, ou même un atome de fluor.

Dans le deuxième type de formule générale, le radical X peut être un radical hydrocarboné, substitué par un ou plusieurs groupements fonctionnels, relié au silicium (liaison A ou D) par au plus une liaison non hydrolysable.

Une caractéristique importante du procédé selon l'invention est que l'hydrolyse du précurseur ait lieu dans des conditions "douces", c'est à dire avec un pH neutre et en l'absence de tout catalyseur chimique ou enzymatique. Le précurseur est hydrolysé dans un solvant contenant de 0,1% à 5% d'eau par rapport à l'ensemble. De préférence, le précurseur est ajouté au solvant (contenant l'eau) goutte à goutte, sous agitation, en contrôlant continuellement la température. Le solvant peut être un alcool tel que l'alcool éthylique, l'isopropanol ou un alcool gras tel que le cyclohexanol ou l'octyldodécanol, un glycol tel que le propylène glycol, le butylène glycol, l'hexylène glycol ou le polyéthylène-glycol (ex: PEG 400), ou bien encore un solvant organique miscible avec l'eau tel que l'acétone ou l'acétate d'éthyle. Lorsque l'hydrolyse libère un acide, il est judicieux d'ajouter une quantité stoechiométrique de base compatible avec le solvant utilisé, telle que la triéthanolamine, afin de neutraliser l'acide formé dans l'hydrolyse. Lorsque l'hydrolyse libère une base, il peut être préférable d'ajouter une quantité stoechiométrique d'acide. Les acides convenables sont de préférence dès acides carboxyliques, et en particulier l'acide acétique ou l'acide lactique.

Une alternative à la méthode ci-dessus consiste à solubiliser d'abord le précurseur dans un milieu huileux contenant une faible quantité d'eau comme défini précèdemment. On ajoute ensuite une faible quantité d'un alcool ou autre glycol miscible avec l'huile. La composition obtenue peut être utilisée pour former une émulsion.

Une autre caractéristique essentielle est que l'atome de silicium est lié par au moins une liaison hydrolysable à un composé dit stabilisant. Après hydrolyse, lorsque le précurseur est selon le premier type de la formule générale, l'agent stabilisant est libéré dans le milieu et stabilise le silyl en créant des liaisons faibles avec lui (ponts hydrogènes) et empêche la polycondensation. Le pouvoir stabilisant de ces agents peut aussi s'expliquer par leur capacité à reformer une liaison covalente transistoire. On obtient alors une structure dynamique où certaines liaisons possèdent un "caractère mixte" (liaison hydrogène, liaison covalente). Bien sûr, lorsque le précurseur est selon le deuxième type de la formule générale, le stabilisant peut rester lié au silyl par une liaison covalente.

Les stabilisants répondant au critère énoncé ci-dessus sont, après hydrolyse, des composés hydroxyacides carboxyliques et particulièrement les alpha et les bêta hydroxyacides, les glucuronides, des composés possédant plusieurs fonctions alcools (ou phénols) et surtout des fonctions alcools (ou phénols) vicinales. On peut citer dans cette catégorie les glycols, les catéchols et les catécholamines (DOPA, adrénaline), les polyéthylèneglycols, les polyols tel que le glycérol, les monosaccharides (L-thréose, L-ribose, sorbitol...) ; des acides phénoliques tels que l'acide gallique, l'acide 3,4-dihydroxybenzoique ou l'acide cafféique, et dérivés estérifiés ; les diacides tels que l'acide malonique ; certains composés possédant une géométrie particulière propre à stabiliser en milieu aqueux les complexes de silyls, telles que les tropolones (ex: la thujaplicine).

Des exemples de précurseurs pouvant être utilisés dans le procédé selon l'invention sont donnés ci-après :

### Dérivés d'acides salicyliques :

### 2,2-éthoxyméthyl-4-oxobenzo-1,3-dioxa-2-silane

*Stabilisant:* acide salicylique

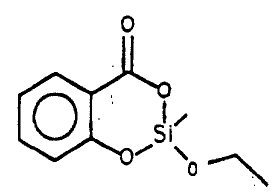

### 2,2-diméthyl-4-oxobenzo-1,3-dioxa-2-silane

*Stabilisant:* acide salicylique

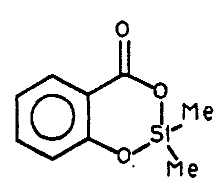

### 2,2-éthoxy, n-octyl-4-oxobenzo-1,3-dioxa-2-silane

*Stabilisant*: acide salicylique

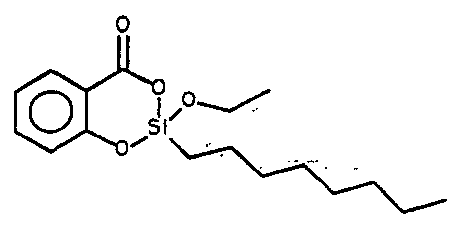

### Divers :

### 1,2-3,4-5,6-éthoxyméthylsilyl-sorbitol ou tri(éthoxyméthylsilyl)sorbitol

*Stabilisant:* acide sorbitol

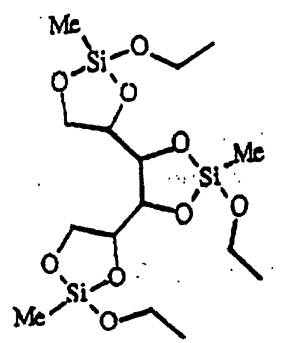

### 2,3-diméthyldilyloxy-N-(3-hydroxypropyl)-3,3-butanamide ou diméthylsilylpantothénol :

*Stabilisant:* panthénol

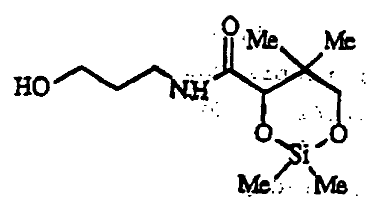

### Bis-diméthylsilyl-ascorbate :

Stabilisant: acide ascorbique

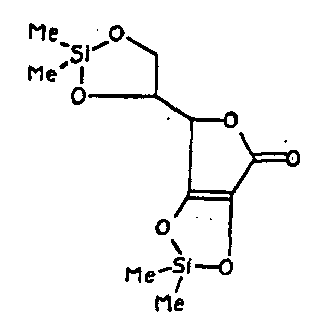

### 2,2-éthoxyméthyl-4-oxo-5-méthyl-1,3-dioxa-2-cyclosilane :

*Stabilisant:* acide lactique

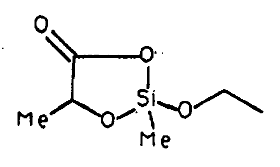

### Caproyl-diéthoxyméthylsilane

*Stabilisant:* acide caproïque

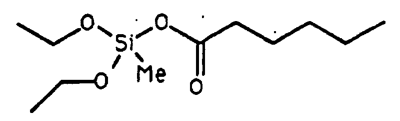

De façon générale, les composés selon l'invention pourront être utilisés dans de nombreuses applications mettant en jeu les propriétés des formes de "silyl", à savoir les propriétés thérapeutiques, diététiques ou cosmétiques découlant de leur activité anti-inflammatoire, régénératrice, anti-dégénérescente, normalisatrice, stimulatrice métabolique, anti-radicalaire et anti-glycation, et de façon générale l'activité de stimulation des défenses de l'organisme.

L'intérêt des produits obtenus par le procédé selon l'invention est qu'ils présentent une forte concentration en silyl. Alors que la plus forte concentration pouvant être obtenue par les méthodes classiques sans risquer la polycondensation est de 2g par litre d'eau, il est possible d'obtenir, en utilisant le procédé de l'invention, une concentration en silyl allant jusqu'à 50g par kg de solvant.

Les solutions concentrées de silyl peuvent être préparées en utilisant divers solvants, ce qui offre une grande souplesse pour leur formulation. Elles peuvent donner lieu à différentes formulations de type aqueux ou semi-aqueux telles que des lotions, collyres, solutions pour bains de bouche, crèmes, gels, dentifrices, chewingum, etc... Les concentrés huileux de silyl sont préférablement utilisés pour la préparation d'émulsions ou de crèmes. Lorsque les silyls concentrés sont préparés dans un solvant relativement volatil (acétone, éthanol), il est possible, après formulation dans un solvant moins volatil (tel que l'eau) d'éliminer le solvant d'origine des silyls. Ceci est réalisé généralement par évaporation sous pression réduite.

Les exemples qui suivent sont illustratifs (mais non limitatifs) des formulations utilisées comme médicaments ou produits cosmétiques pour mettre en évidence les activités énoncées ci-dessus.

### EXEMPLE 1 :

### Activité régénératrice

### Restructuration des tuniques capillaires

Dans cet exemple, le précurseur hydrolysé selon le procédé de l'invention est le Caproyl diéthoxyméthylsilane, de M.M. = 248,39, soit en SiOH = 19%, ce qui équivaut en une concentration en Si égale à 30g/Kg.

A partir du silyl obtenu, on formule un gel aqueux de Carbopol protégé par du propyl- et méthyl-paraben (on élimine le solvant de départ par évaporation sous pression réduite). On obtient ainsi une formulation à très forte concentration de principe actif égale à 5,3%.

Ce gel a été testé sur des lymphoedèmes du membre supérieur par pressothérapie séquentielle et drainages lymphatiques sur 62 patientes par des masseurs kinésithérapeutes.

Les résultats ont été les suivants en terme de diminution du colume d'oedème:

| Nombre de patientes | Diminution |
|---|---|
| 3 | < 10% |
| 13 | de 10 à 25% |
| 29 | de 25 à 50% |
| 13 | de 50 à 75% |
| 2 | de 75 à 100% |
| 2 | ≥ 100% |

ce qui donne une moyenne de diminution de 45 %

### EXEMPLE 2 :

### Activité anti-inflammatoire hydratante régénératrice

### Composition dermo-pharmaceutique

Dans cet exemple on a utilisé comme précurseur du 2,2-éthoxy-n-octyl-4-oxobenzo-1,3-dioxa-2-silane ayant pour formule C₁₇H₂₆O₄Si de M.M. = 322,48, correspondant à 14% de SiOH après hydrolyse. On utilise la solution concentrée résultante dans le stéaryloctanoate additionné d'éthanol.

Le n-octyl-silyl-salicylate obtenu est utilisé comme produit cosméto-dermopharmaceutique destiné à des peaux dénutries ou perturbées par le soleil, le vent, l'achné...

Des tests ont porté sur une lotion astreingeante, régénératrice, anti-inflammatoire, hydratante.

| | |
|---|---|
| Sulfocarbonate de zinc | 3,00 |
| Stéaryl octanoate | 8,00 |
| n-octyl-silyl-salicylate | 3,50 |
| Alcool éthylique | 20,00 |
| Eau déminéralisée | 100,00 |

Sur le plan de l'activité biologique, il faut noter que les alcools gras ramollissent les amas de kératine et que l'acide salicylique libéré au niveau cutané contribue à les dissocier. La fonction SiOH augmente l'action de l'acide salicylique en assurant une pénétration plus importante et normalise le métabolisme des kératinocytes et leur prolfération. L'inflammation regresse avec une cicatrisation correcte des boutons.

### EXEMPLE 3 :

### Action anti-inflammatoire

### Thérapie stomatologique

Pour cette composition, on a utilisé comme précurseur du 2,2-diméthyl-4-oxobenzo-1,3-dioxa-2-silane de formule C₁₀H₁₂O₃Si et M.M. = 194,26, ce qui équivaut à 24% de SiOH après hydrolyse, ou du 2,2-éthoxyméthyl-4-oxobenzo-1,3-dioxa-2-silane de formule C₁₀H₁₂O₄Si et M.M. = 224,26, ce qui équivaut à 21% SiOH après hydrolyse. L'hydrolyse ménagée est réalisée dans un solvant approprié avant formulation.

Plusieurs produits d'hygiène gingivaux ont été testés: bains de bouche, dentifrice et chewing-gum, et la liberté a été laissée aux personnes impliqués dans ces essais. Dans plus de 70% des cas, on a observé une action anti-inflammatoire et régénératrice de la gencive pour tout le groupe, et pour deux personnes qui ont continué l'utilisation du gel gingival à raison de 2 applications par jour, sur plus de 6 mois, ont vu une réduction du déchaussement de leurs dents; cette pathologie étant associée aux désordres ostéoporotiques.

Les silyl-salicylates utilisés dans cet exemple permettent donc de lutter contre les maladies parodontales qui touchent les tissus de soutien des dents et d'assurer ainsi une bonne calcification.

Dans tous les cas, on a noté que, lors de l'utilisation de ces composés, la diminution des saignements spontanés lors du brossage et la réduction de la sensibilité gingivale. Dans un cas, on a noté le recouvrement gingival de certaines dents déchaussées, et dans trois autres cas, on a noté une stabilisation ou un ralentissement du déchaussement.

| Solution gingivale- bain de bouche | |
|---|---|
| Silyl-salicylate | 5,00 |
| Ethanol | 10,00 |
| Essence de menthe | 0,50 |
| eau déminéralisée q.s.p | 100,00 |

| Gel gingival | |
|---|---|
| Carbopol | 0,20 |
| Silyl-salicylate | 5,00 |
| Ethanol | 10,00 |
| Essence de menthe | 0,80 |
| Eau déminéralisée q.s.p | 100,00 |

| Dentifrice | |
|---|---|
| Silyl-salicylate | 4,00 |
| Métaphosphate insoluble | 40,00 |
| Glycérine | 13,00 |
| Sorbitol à 70% | 19,00 |
| Alginate de sodium | 2,00 |
| Laurylsarcossinate de sodium | 2,00 |
| Oxyde de titane | 0,95 |
| Saccharine | 0,20 |
| Essence de menthe | 0,80 |
| Eau déminéralisée q.s.p | 100,00 |

| Chewing-gum | |
|---|---|
| Silyl-salicylate | 1,80 |
| Pâte q.s.p | 100,00 |

### EXEMPLE 4 :

### Action stimulatrice métabolique

### Composition antichute capillaire

Le composé utilisé est le 2,3-diméthylsilyloxy-N-(3-hydroxypropyl)-3,3-butanamide de formule C₁₁H₂₃NO₄ et M.M. = 261,39, ce qui équivaut à 18% de SiOH ou 10,7% en Si.

Le silyl-panthénol obtenu après hydrolyse peut être utilisé soit à l'état pur, soit dilué dans un mélange de butanediol eau 50:50 à raison de 40% de produit pur. Ceci correspond à une concentration en Si de 4%, c'est à dire un apport quantitatif important.

La formule de cette lotion capillaire est la suivante:

| | |
|---|---|
| Silyl panthénol | 5,00 g |
| Butanediol eau 50/50 | 7,50 g |
| Propylène glycol | 14,00 g |
| Alcool à 70° | 100,00 g |

La lotion, appliquée en friction quotidienne, a été testée sur des alopécies séborrhéiques masculines. Sur les 23 sujets testés, 10 patients avaient entre 15 et 25 ans, 11 entre 26 et 35 ans, 1 de 45 ans, et 1 de 55 ans.

Les motis d'inclusion dans l'expérimentation étaient dans tous les cas une chute de cheveux considérée comme anormale, déterminée par une alpécie séborrhéique.

Dans tous les cas, on a constaté un éclaircissement de la chevelure, visible et indiscutable. Les résultats ont été les suivants: *excellents* résultats: 59%, *bons* résultats: 9%, *moyens:* résultats 18%, et résultats *nuls:* 14%.

Les résultas ont été confirmés par des trichogrammes faits pratiquement à un an d'intervalle, en déterminant le rapport anagènes/télogènes. Ils ont été les suivants:
- avant expérimentation rapports A/T - frontal 2,8 - occipital 1,5 - temporal 4
- après expérimentation rapport A/T - frontal 4,8 - occipital 3,6 - temporal 5

La moyenne obtenus sur les meilleurs résultats (excellents + bons) a donc été de 70% des sujets traités.

### EXEMPLE 5 :

### Action régénératrice

### Composition cosmétique anti-rides

Différents dérivés α-hydroxy-acides silyls sont été formulés, à partir des précurseurs diméthylsilyl-2-oxo-octanoate ou éthoxyméthylsilyl-2-oxo-octanoate ou 2,2-éthoxyméthyl-4-oxo-5-méthyl-1,3-dioxa-2-cyclosilane en solution à 30% dans du 1,3 butanediol, ce qui correspond en moyenne et en solution à des apports en SiOH de 8%, soit 5% en Si.

Il est également possible d'utiliser comme précurseurs des dérivés d'amino-acides tels que le Diméthyl-N-acétyl-cystéine (M.M. = 219,33) en solution à 60% dans de l'octyldodécanol, ce qui correspond à un apport en SiOH de 14%.

Les différents silyls obtenus après hydrolyse ont été utilisés dans des formules de crème, lait, gel, ou lotion, à des doses de 5 à 10%, ce qui donnes des apports en SiOH de 0,7 à 1,4%.

Ainsi, on a réalisé un gel aqueux contenant 7,6% de silyl-2-hydroxycaprilate en utilisant du 1,3-butanediol (30:70) qui correspond à un apport de 0,50% de SiOH, soit 3 fois la teneur du même produit obtenu sans le procédé de l'invention. Le gel a été appliqué quotidiennement le soir sur une peau parfaitement nettoyée.

Les personnes testées étaient des femmes de 55 à 67 ans. On a observé une réduction des ridules et une atténuation des rides profondes. La peau se trouvait plus lisse, plus lumineuse et plus tonique, comme cela a été vérifié par des tests in vitro, sur des cultures de cellules de fibroblastes vieillis.

Une recherche d'activité cytostimulatrice a été menée sur des cultures monocouches de kératinocytes et de fibroblastes. la prolifération cellulaire a été mesurée par un test colorimétrique au rouge neutre. L'analyse a été faite en spectrométrie UV (540nm). Pour simuler les conditions d'un test avec des cellules vieillies, ces dernières ont été cultivées dans un milieu déprimant contenant des concentrations de sérum de veau foetal (SVF) suboptimales.

*Résultats*: la croissance des cellules a été augmentée de façon significative après enrichissement du milieu de culture par des solutions de silyls concentrées. L'amplitude de réponse à la cytostimulation a été très importante, particulièrement sur des fibroblastes, cellules impliquées dans la synthèse du collagène et de l'élastine, qui ont exprimé une réaction très marquée. On a constaté une stimulation de 200% de la cinétique de prolifération fibroblastique par rapport à des témoins cultivés en présence de 2,5% de SVF.

### EXEMPLE 6 :

### Activité normalisatrice

### Composition cosmétique hydratante

Dans cet exemple le précurseur choisi a été le tri-éthoxy-méthylsilyl-sorbitol de formule C₁₅H₃₂O₉Si₃, M.M. = 440,67, ce qui équivaut à 31% de SiOH (soit 19% en Si) après hydrolyse.

Avec le silyl-sorbitol obtenu, on a réalisé une émulsion très fine hydrodispersible avec une teneur en lipide de 35%, le silyl-sorbitol étant intégré à 0,5%.

Cette émulsion a été utilisée sur des peaux sèches déshydratées. Dès les premières applications, on a noté une modification superficielle épidermique très sensible au toucher. Il y a rétablissement du film hydrolipidique au niveau épiderme/couche cornée.

Un contôle in vivo par spectre I.R. à transformée de Fourier a mis en évidence une hydratation biologique par apport d'eau liée.

### EXEMPLE 7 :

### Activité anti-radicalaire

### Produits cosmétiques de jour colorés ou pas.

Le précurseur utilisé a été le diméthyl-silyl-2,3,5,6-ascorbate ayant pour formule C₁₀H₁₆O₆Si₂ de M.M. = 288,4, ce qui équivaut à 32% de SiOH (soit 19,4% de Si) après hydrolyse.

Pour apporter une protection du visage vis à vis des phénomènes oxydatifs, on a formulé une crème de jour, émulsion hydrodispersible ou fond de teint dans laquelle le silyl a été incorporé à raison de 1%. Par conséquent, aucune modification n'était à craindre au niveau de la texture des produits, bien que l'on disposait de 0,3% de SiOH (soit 0,19% de Si).

L'activité anti-radicalaire de la formulation a été mise en évidence in vitro par un test consistant à produire des radicaux libres oxygénés par voie enzymatique (action de la xanthine oxydase sur l'acétaldéhyde) et à comparer la résistance des cellules cultivées en présence ou en l'absence de silyl au contact de ces radicaux.

L'addition de radicaux libres au sein d'une culture cellulaire induit une cytotoxicité. cette toxicité se traduit par une lyse cellulaire avec une augmentation de lactate déshydrogénase (LDH) cellulaire. L'évaluation de la résistance cellulaire au stress radicalaire est obtenue par le dosage de l'activité de la LDH en spectrophotométrie UV.

*Résultats*: On a observé un effet très sensible de la formulation utilisée. Le système peroxydatif utilisé a produit des ions superoxydes et du peroxyde d'hydrogène. L'étude des résultats montre que la formulation induit une protection contre la lyse cellulaire spontanée (activité de la LDH diminuée de 67%). Cette protection persiste et s'intensifie nettement (diminution de la LDH de 75%) en présence d'un stress radicalaire provoqué.

## Revendications

1. Procédé de préparation d'un mélange de composés à base de silicium biologiquement actif consistant à hydrolyser un précurseur de formule générale: 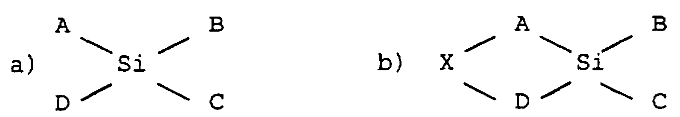 dans laquelle A, B, C, D peuvent être de l'hydrogène, et dans laquelle A, B, C, D et X désignent des radicaux différents de OH et 2 ou 3 de ces radicaux sont hydrolysables ; les radicaux qui sont hydrolysables étant des radicaux liés à Si par un atome d'oxygène ou par un atome d'azote ou de soufre, notamment des esters ou des radicaux alkoxy, aryloxy tels que des radicaux phénoxy ou des radicaux allyloxy ou vinyloxy lorsque l'atome de silicium est lié directement à un atome d'oxygène; soit des thioesters ou des radicaux aryl ou alkylthioéthers lorsque le silicium est lié par un atome de soufre; soit des amines mono ou disubstituées par des chaînes hydrocarbonées substituées ou non par un ou plusieurs groupements fonctionnels, des aryl ou alkylamides lorsque le silicium est lié par un atome d'azote ; les radicaux qui ne sont pas hydrolysables étant des radicaux hydrocarbonés, substitués ou non par un ou plusieurs groupements fonctionnels, certains radicaux alkoxy stériquement encombrés, les radicaux fluorocarbonés, ou même un atome de fluor ; et X étant un radical hydrocarboné, substitué par un ou plusieurs groupements fonctionnels, relié au silicium (liaison A ou D) par au plus une liaison non hydrolysable ;
l'hydrolyse étant effectuée dans un solvant contenant une faible quantité d'eau dans une proportion par rapport au solvant comprise de préférence entre 0,1% et 5% ; et
au moins un des composés provenant des liaisons hydrolysées du silicium étant un composé dit stabilisant empêchant la formation de polymères à partir des liaisons hydrolysées du silicium.

2. Procédé selon la revendication 1 effectué avec un pH neutre et en l'absence de tout catalyseur chimique ou enzymatique, et dans lequel ledit précurseur est ajouté goutte à goutte audit solvant contenant une faible quantité d'eau, sous agitation, en contôlant continuellemnt la température.

3. Procédé selon la revendication 1 ou 2, dans lequel ledit solvant est choisi dans le groupe consistant en alcools tels que l'alcool éthylique, l'isopropanol ou un alcool gras tel que le cyclohexanol ou l'octyldodécanol, glycols tels que le propylène glycol, le butylène glycol, l'hexylène glycol ou le polyéthylène glycol, et les solvants organiques miscibles avec l'eau tels que l'acétate d'éthyle ou l'acétone.

4. Procédé selon la revendication 1 consistant à solubiliser d'abord ledit précurseur dans un composé huileux contenant une faible quantité d'eau, et d'ajouter ensuite une faible quantité d'un alcool ou glycol miscible avec ledit composé huileux.

5. Application d'un composé obtenu par le procédé selon l'une des revendications 1 à 4, pour obtenir une composition thérapeutique, diététique ou cosmétique ayant une activité anti-inflammatoire, régénératrice, anti-dégénérescente, normalisatrice, stimulatrice métabolique, anti-radicalaire et anti-glycation, et de façon générale une activité de stimulation des défenses de l'organisme humain ou animal.

6. Composition thérapeutique comportant un composé obtenu par le procédé selon l'une des revendications 1 à 4 dans lequel ledit précurseur est du caproyl-diéthoxyméthylsilane, ladite composition thérapeutique ayant une activité régénératrice pour la restructuration des tuniques capillaires.

7. Composition thérapeutique selon la revendication 6 dans laquelle le composé est administré sous forme de gel aqueux.

8. Composition thérapeutique comportant un composé obtenu par le procédé selon l'une des revendications 1 à 4 dans lequel ledit précurseur est du 2,2-éthoxy-n-octyl-4-oxobenzo-1,3-dioxa-2-silane, ladite composition thérapeutique ayant une activité anti-inflammatoire, hydratante et régénératrice pour le traitement des pathologies cutanées.

9. Composition thérapeutique comportant un composé obtenu par le procédé selon l'une des revendications 1 à 4 dans lequel ledit précurseur est du 2,2-diméthyl-4-oxobenzo-1,3-dioxa-2-silane ou du 2,2-éthoxyméthyl-4-oxobenzo-1,3-dioxa-2-silane, ladite composition thérapeutique ayant une action anti-inflammatoire pour la thérapie stomatologique.

10. Composition thérapeutique selon la revendication 9 dans laquelle le composé est utilisé sous forme de solution gingivale, gel gingival, dentifrice ou chewingum.

11. Composition thérapeutique comportant un composé obtenu par le procédé selon l'une des revendications 1 à 4 dans lequel ledit précurseur est du 2,3-diméthylsilyloxy-N-(3-hydroxypropyl)-3,3-butanamide, ladite composition thérapeutique ayant une activité stimulatrice métabolique pour le traitement contre la chute des cheveux.

12. Composition thérapeutique selon la revendication 11 dans laquelle le composé est utilisé sous forme de lotion capillaire.

13. Utilisation d'un composé obtenu par le procédé selon l'une des revendications 1 à 4 dans lequel ledit précurseur est un dérivé α-hydroxy-acide silyl choisi dans le groupe formé de diméthylsilyl-2-oxo-octanoate, éthoxyméthylsilyl-2-oxo-octanoate, ou 2,2-éthoxyméthyl-4-oxo-5-méthyl-1,3-dioxa-2-cyclosilane, pour le traitement cosmétique anti-rides.

14. Composition cosmétique comportant un composé obtenu par le procédé selon l'une des revendications 1 à 4 dans lequel ledit précurseur est du tri-éthoxy-méthylsilyl-sorbitol, ladite composition cosmétique ayant une activité normalisatrice pour le traitement contre les peaux sèches

15. Utilisation cosmétique d'un composé obtenu par le procédé selon l'une des revendications 1 à 4 dans lequel ledit précurseur est du diméthyl-silyl-2,3,5,6-ascorbate, pour la protection de la peau.

## Patentansprüche

1. Verfahren zur Herstellung einer Mischung von Verbindungen auf der Basis von biologisch aktivem Silicium, das darin besteht, dass ein Vorläufer der allgemeinen Formel 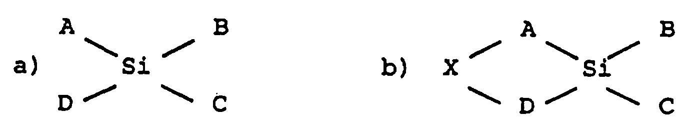 hydrolysiert wird, in der A, B, C, D Wasserstoff sein können und in der A, B, C, D und X von OH verschiedene Reste bezeichnen und 2 oder 3 dieser Reste hydrolysierbar sind; wobei die hydrolysierbaren Reste Reste sind, die an Si durch ein Sauerstoffatom oder durch ein Stickstoff- oder Schwefelatom gebunden sind, und zwar insbesondere Ester oder Alkoxy-, Aryloxy-Reste, wie z.B. Phenoxy-Reste, oder Allyloxy- oder Vinyloxy-Reste, wenn das Siliciumatom direkt an ein Sauerstoffatom gebunden ist; oder Thioester oder Aryl-Reste oder Alkylthioether, wenn das Silicium durch ein Schwefelatom gebunden ist; oder Amine, die durch Kohlenwasserstoffketten mono- oder disubstituiert sind, die durch eine oder mehrere funktionelle Gruppen substituiert sind oder nicht, Aryl- oder Alkylamide, wenn das Silicium durch ein Stickstoffatom gebunden ist; wobei die Reste, die nicht hydrolysierbar sind, durch eine oder mehrere funktionelle Gruppen substituierte Kohlenwasserstoffreste, gewisse sterisch behinderte Alkoxy-Reste, Fluorkohlenstoffreste oder auch ein Fluoratom sind;
und X ein durch eine oder mehrere funktionelle Gruppen substituierter Kohlenwasserstoffrest ist, der an das Silicium (Bindung A oder C) durch höchstens eine nicht hydrolysierbare Bindung gebunden ist;
wobei die Hydrolyse in einem Lösungsmittel durchgeführt wird, das eine kleine Menge Wasser in einem Anteil von vorzugsweise 0,1% bis 5%, bezogen auf das Lösungsmittel, enthält;
und wenigstens eine der von den hydrolysierten Bindungen des Siliciums stammenden Verbindungen eine sogenannte stabilisierende Verbindung ist, die die Bildung von Polymeren aus den hydrolysierten Bindungen des Siliciums verhindert.

2. Verfahren nach Anspruch 1, das mit einem neutralen pH und ohne jeden chemischen oder enzymatischen Katalysator durchgeführt wird und bei dem der Vorläufer dem eine kleine Menge Wasser enthaltenden Lösungsmittel tropfenweise unter Rühren beigegeben wird, indem die Temperatur ständig gesteuert wird.

3. Verfahren nach Anspruch 1 oder 2, bei dem das Lösungsmittel aus der Gruppe ausgewählt ist, die aus Alkoholen, wie z.B. Ethylalkohol, Isopropanol oder einem Fettalkohol, wie z.B. Cyclohexanol oder Octyldodecanol, Glykolen, wie z.B. Propylenglykol, Butylenglykol, Hexylenglykol oder Polyethylenglykol, und den mit Wasser mischbaren organischen Lösungsmitteln, wie z.B. Ethylacetat oder Aceton, besteht.

4. Verfahren nach Anspruch 1, das darin besteht, dass zunächst der Vorläufer in einer öligen Verbindung, die eine kleine Menge Wasser enthält, solubilisiert wird und dann eine kleine Menge eines Alkohols oder Glykols beigegeben wird, der bzw. das mit der öligen Verbindung mischbar ist.

5. Anwendung einer mit dem Verfahren nach einem der Ansprüche 1 bis 4 erhaltenen Verbindung zur Herstellung einer therapeutischen, diätetischen oder kosmetischen Zusammensetzung mit einer entzündungshemmenden, regenerierenden, antidegenerierenden, normalisierenden, stoffwechselstimulierenden Wirkung, mit einer Wirkung gegen freie Radikale und mit Antiglykationswirkung und allgemein mit einer die Abwehrkräfte des menschlichen oder tierischen Organismus stimulierenden Wirkung.

6. Therapeutische Verbindung, die eine mit dem Verfahren nach einem der Ansprüche 1 bis 4 hergestellte Verbindung enthält, bei der der Vorläufer Caproyldiethoxymethylsilan ist, wobei diese therapeutische Zusammensetzung eine regenerierende Wirkung bei der Restrukturierung der Tunica des Haares hat.

7. Therapeutische Zusammensetzung nach Anspruch 6, in der die Verbindung in Form von wässrigem Gel verabreicht wird.

8. Therapeutische Zusammensetzung, die eine mit dem Verfahren nach einem der Ansprüche 1 bis 4 hergestellte Verbindung enthält, in welcher der Vorläufer 2,2-Ethoxy-n-octyl-4-oxobenzo-1,3-dioxa-2-silan ist, wobei diese therapeutische Zusammensetzung eine entzündungshemmende, hydratisierende und regenerierende Wirkung bei der Behandlung von Hautpathologien hat.

9. Therapeutische Zusammensetzung, die eine mit dem Verfahren nach einem der Ansprüche 1 bis 4 hergestellte Verbindung enthält, in der der Vorläufer 2,2-Dimethyl-4-oxobenzo-1,3-dioxa-2-silan oder 2,2-Ethoxymethyl-4-oxobenzo-1,3-dioxa-2-silan ist, wobei diese therapeutische Zusammensetzung eine entzündungshemmende Wirkung bei der stomatologischen Therapie hat.

10. Therapeutische Zusammensetzung nach Anspruch 9, in der die Verbindung in Form von Gingivallösung, Gingivalgel, Zahncreme oder Kaugummi verwendet wird.

11. Therapeutische Zusammensetzung, die eine mit dem Verfahren nach einem der Ansprüche 1 bis 4 hergestellte Verbindung enthält, in der der Vorläufer 2,3-Dimethylsilyloxy-N-(3-hydroxypropyl)-3,3-butanamid ist, wobei diese therapeutische Zusammensetzung eine stoffwechselstimulierende Wirkung bei der Behandlung von Haarausfall hat.

12. Therapeutische Zusammensetzung nach Anspruch 11, in der die Verbindung in Form von Haarwasser verwendet wird.

13. Verwendung einer mit dem Verfahren nach einem der Ansprüche 1 bis 4 hergestellten Verbindung, in der der Vorläufer ein Silyl-α-hydroxysäurederivat ist, das aus der Gruppe ausgewählt ist, die von Dimethylsilyl-2-oxooctanoat, Ethoxymethylsilyl-2-oxooctanoat oder 2,2-Ethoxymethyl-4-oxo-5-methyl-1,3-dioxa-2-cyclosilan gebildet wird, für die kosmetische Behandlung von Falten.

14. Kosmetische Zusammensetzung, die eine mit dem Verfahren nach einem der Ansprüche 1 bis 4 hergestellte Verbindung enthält, in der der Vorläufer Triethoxymethylsilylsorbit ist, wobei diese kosmetische Zusammensetzung eine normalisierende Wirkung bei der Behandlung von trockener Haut hat.

15. Kosmetische Verwendung einer mit dem Verfahren nach einem der Ansprüche 1 bis 4 hergestellten Verbindung, in der der Vorläufer Dimethylsilyl-2,3,5,6-ascorbat ist, für den Schutz der Haut.

## Claims

1. Preparation process of a compound containing biologically active silicon, consisting in hydrolyzing a precursor having the formula: 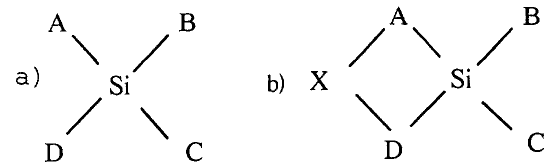 in which A, B, C ,D can be hydrogen and in which A, B, C ,D and X indicate radicals different from OH and 2 or 3 of these radicals are hydrolysable ; the radicals which are hydrolysable being radicals linked to Si by an oxygen atom, a nitrogen atom or by a sulphur atom, notably esters or alkoxy, aryloxy radicals such as phenoxy radicals or allyloxy or vinyloxy radicals when the silicon atom is directly bond to an oxygen atom ; either thioesters or aryl or alkylthioether radicals when the silicon is bound by a sulphur atom ; either amines mono or disubstituted by hydrocarbon chains substituted or not by one or several functional groups, aryl or alkylamides when the silicon is bound to a nitrogen atom; the radicals which are not hydrolysable being hydrocarbon radicals, substituted or not by one or several functional groups, certain alkoxy radicals with a steric hindrance, the fluorocarbon radicals or even a fluorine atom ; and X being an hydrocarbon radical, substituted by one or several functional groups, bound to silicon (bond A or D) by at the best one nonhydrolysable bond ;
the hydrolysis being carried out in a solvent containing a small quantity of water in a proportion with regard to the solvent preferably included between 0.1 % and 5 %, and at least one of these compounds obtained from the hydrolyzed bonds of silicon being a compound said stabilizing and preventing the formation of polymers from the silicon hydrolyzed bonds.

2. Process according to claim 1 performed at neutral pH and in the absence of any chemical or enzymatic catalyst and in which the said precursor is added drop by drop into the solvent containing a small quantity of water, under stirring, and under constant checking of the temperature.

3. Process according to claim 1 or 2, in which the said solvent is chosen in the group consisting in alcohols such as ethyl alcohol, isopropanol or a fatty alcohol such as the cyclohexanol or the octyldodecanol, glycols such as the propylene glycol, the butylene glycol, the hexylene glycol or the polyethylene glycol, and the water miscible organic solvents such as the ethyl acetate or the acetone.

4. Process according to claim 1 consisting in solubilizing first the precursor in an oily compound containing a small quantity of water, and then in adding a small quantity of alcohol or glycol miscible with the said oily compound.

5. Application of a compound obtained by the process according to the claims 1 to 4, in order to obtain a therapeutic, dietetic or cosmetic composition having an anti-inflammatory, regenerating, anti-degeneration, normalizer, metabolic stimulator, anti free-radical and anti-glycation, or generally speaking an activity of stimulation of the human or animal organism defences.

6. Therapeutic composition containing a compound obtained by the process according to the claims 1 to 4 in which the said precursor is the caproyldiethoxymethylsilane, the said therapeutic composition having a regenerating activity for the re-structuration of the capillary coats.

7. Therapeutic composition according to claim 6 in which the compound is administered under the form of an aqueous gel.

8. Therapeutic composition containing a compound obtained by the process according to one of the claims 1 to 4 in which the precursor is the 2,2-ethoxy-n-octyl-4-oxobenzo-1,3-dioxa-2-silane, the said therapeutic composition having an anti-inflammatory, hydrating and regenerating activity for the cutaneous pathologies treatment.

9. Therapeutic composition containing a compound obtained by the process according to one of the claims 1 to 4 in which the said precursor is the 2,2-dimethyl-4-oxobenzo-1,3-dioxa-2-silane or the 2,2-ethoxymethyl-4-oxobenzo-1,3-dioxa-2-silane, the said therapeutic composition having an anti-inflammatory action for the stomatologic therapy.

10. Therapeutic composition according to the claim 9, in which the compound is used under the form of a gingival solution, gingival gel, tooth-paste or chewing gum.

11. Therapeutic composition containing a compound obtained by the process according to one of the claims 1 to 4 in which the said precursor is the 2,3-dimethylsilyloxy-N-(3-hydroxypropyl)-3,3-butanamide, the said therapeutic composition having a metabolic stimulating activity for the hair loss treatment.

12. Therapeutic composition according to claim 11 in which the compound is used under the form a hair lotion.

13. Use of a compound obtained by the process according to one of the claims 1 to 4 in which the said precursor is a derivative of silyl alpha-hydroxy-acid chosen in the group formed by the dimethylsilyl-2-oxo-octanoate, ethoxymethylsilyl-2-oxo-octanoate, or the 2,2 ethoxymethyl-4-oxo-5-methyl-1,3-dioxa-2-cyclosilane, for the anti-wrinkles cosmetic treatment.

14. Cosmetic composition containing a compound obtained by the process of one of the claims 1 to 4 in which the said precursor is the tri-ethoxy-methylsilyl-sorbitol, the said cosmetic composition having a normalizing activity for the dry skins treatment.

15. Cosmetic use of a compound obtained by the process according to one of the claims 1 to 4 in which the said precursor is the dimethyl-silyl-2,3,5,6-ascorbate, for the skin protection.
